# EUROPEAN PATENT APPLICATION

(11) **EP 4 531 058 A1**
(43) Date of publication of application: **02.04.2025**
(21) Application number: 23200330.1
(22) Date of filing: 28.09.2023
(51) Int. Cl.: G16H 80/00, G16H 40/67, G16H 30/20, A61B 34/20, A61B 34/00, A61B 90/00

(54) **VISUALIZATION FOR MEDICAL INTERVENTIONS**

(71) Applicant: Koninklijke Philips N.V., 5656 AG Eindhoven (NL)
(72) Inventor: ELENBAAS, Thijs, Eindhoven (NL); KOSSEN, Rebecca, Eindhoven (NL); NETO FONSECA, Lucia Teresa, 5656AG Eindhoven (NL); SPIJKERBOER, Koen, Eindhoven (NL)
(74) Representative: Philips Intellectual Property & Standards

(57) **Abstract**

The present invention relates to visualization for medical interventions. In order to provide facilitated ways of capturing the user's view, a visualization arrangement (10) for medical interventions is provided. The arrangement comprises a camera device (12) with at least one camera (14) and a view tracking device (16) with at least one tracker. The view tracking device is configured to track a first user's viewing direction (18). The camera device is configured to provide a live stream of images of a scenery of interest with an adaptable camera viewing direction (20). The camera device is configured to adapt the camera viewing direction to be aligned with the tracked first user's viewing direction. The camera device is configured to provide the data of the live stream of images for presentation to a second user or more.

## Description

### FIELD OF THE INVENTION

The present invention relates to a visualization arrangement for medical interventions, to a medical visualization system and to a method for providing additional visual information during a medical intervention.

### BACKGROUND OF THE INVENTION

During medical interventions, such as surgery procedures, sharing information among the staff members has been shown to be of value. While sensor data of the patient can be easily shared with other staff members, the sharing of a current situation in a region of interest of the subject is usually restricted to those around the operation table. In the context of augmented reality appliances, cameras are provided integrated in head-mounted devices that capture the scenery in front of the user. However, it has been shown that such head-mounted devices are complex and may be cumbersome to wear.

### SUMMARY OF THE INVENTION

There may thus be a need to provide facilitated ways of capturing the user's view.

The object of the present invention is solved by the subject-matter of the independent claims; further embodiments are incorporated in the dependent claims. It should be noted that the following described aspects of the invention apply also for the visualization arrangement for medical interventions, for the medical visualization system and for the method for providing additional visual information during a medical intervention.

According to the present invention, a visualization arrangement for medical interventions is provided. The arrangement comprises a camera device with at least one camera and a view tracking device with at least one tracker. The view tracking device is configured to track a first user's viewing direction. The camera device is configured to provide a live stream of images of a scenery of interest with an adaptable camera viewing direction. The camera device is configured to adapt the camera viewing direction to be aligned with the tracked first user's viewing direction. The camera device is configured to provide the data of the live stream of images for presentation to a second user or more.

As an advantage, the user's point of view can be shared e.g. in a remote collaboration solution where others are able to see the situation so-to-speak from the perspective of the treating physician.

According to an example, the camera device is configured to adapt the camara viewing direction such that the live stream of images provided by the camera device to the second user at least partly shows what the first user is viewing.

According to an example, a data processing unit is provided that is configured to provide a control signal to adapt the camara viewing direction to be aligned with the tracked first user's viewing direction.

According to an example, the view tracking device is camera-based and comprises at least one tracking camera.

According to an example, the view tracking device comprises at least one head-tracker unit. The head-tracker unit is configured to track a spatial position and spatial orientation of the first user's head for determining a possible viewing direction of the first user.

According to an example, the view tracking device comprises at least one eye-tracker unit. The eye-tracker unit is configured to track a spatial position and spatial orientation of the first user's pair of eyes for determining a possible viewing direction of the first user.

In an option, a filtering unit is provided for eye-tracking with filtering.

According to an example, the camera device comprises at least one movable camera.

According to an example, the camera device comprises a plurality of fixed cameras.

According to an example, an algorithm is provided that translates the first user's viewing direction to an over-the-shoulder viewing position and vector direction for the provision of the live stream of images. The camera viewing direction is adapted to best match the over-the-shoulder viewing position and vector direction.

According to an example, a focus of the first user's view is detected, and a focus indicator is projected overlaid to the live stream of images. Upon detecting a change in the viewing direction and thus a change of the focus, the focus indicator is adapted in its relative location first and the change of the camera viewing direction is adapted with a temporal delay.

According to the present invention, also a medical visualization system is provided. The system comprises a visualization arrangement according to one of the preceding examples and a display arrangement comprising at least one display device. The at least one display device is configured to present the live stream of images to at least one second user based on the provided data of the live stream of images.

According to the present invention, also a method for providing additional visual information during a medical intervention is provided. The method comprises the steps of:
- Tracking a first user's viewing direction with a view tracking device with at least one tracker;
- Capturing, by a camera device with at least one camera, a live stream of images of a scenery of interest; the camera device is adapting the camara viewing direction to be aligned with the tracked first user's viewing direction; and
- Presenting the live stream of images to at least one second user.

According to an aspect, head and eyes of the treating physician are tracked to determine the direction of viewing. Using a movable, rotatable camera, a 3rd-person-look-over-the-shoulder-view is generated. Due to relying on a camera mounted to a fixed structure, i.e. not to user, the view can be provided as a motion-stabilized view. In an example, an affordable, low-load robotic arm functions as a platform for translating and tilting of the camera.

According to an aspect, head and eyes of the treating physician are tracked to determine the direction of viewing. Using a movable, rotatable camera, a (motion-stabilized) 3rd-person-look-over-the-shoulder-view is generated like those of some computer games. This has the advantage of providing an image stream from the perspective of the user.

In a minimal embodiment, the camera only rotates without translation. In an alternative embodiment, no movable camera is used but instead, a panel containing a matrix of cheap, e.g. 360-degree, cameras are used, where the optimal camera is selected.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows an example of a visualization arrangement for medical interventions in the context of an example of a medical visualization system.
Fig. 2 shows basic steps of an example of a method for providing additional visual information during a medical intervention.

### DETAILED DESCRIPTION OF EMBODIMENTS

Certain embodiments will now be described in greater details with reference to the accompanying drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

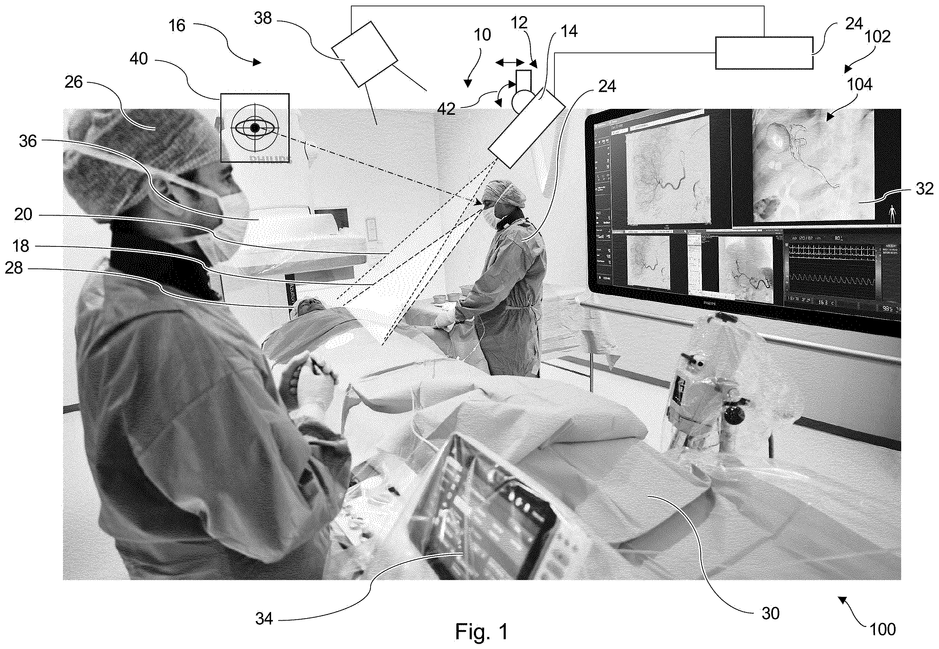

Fig. 1 shows an example of a visualization arrangement 10 for medical interventions in the context of an example of a medical visualization system. The arrangement 10 comprises a camera device 12 with at least one camera 14. The arrangement 10 also comprises a view tracking device 16 with at least one tracker. The view tracking device 16 is configured to track a first user's viewing direction 18. The camera device 12 is configured to provide a live stream of images of a scenery of interest with an adaptable camera viewing direction 20. The camera device 12 is configured to adapt the camera viewing direction 20 to be aligned with the tracked first user's viewing direction 18. The camera device 12 is configured to provide the data of the live stream of images for presentation to a second user or more.

The first user, indicated with reference numeral 24, is shown in the center background of Fig. 1; the second user, indicated with reference numeral 26, is shown in the left foreground of Fig. 1. A subject 28 is indicated as laying on a subject support 30.

A main monitor arrangement 32 is shown as an option in the right part; further, a table-side graphical interface 34 is provided as an option for the second user 26. A C-arm imaging system 36 is indicated in the background.

The term "to align" refers to a best matching of the field of view, i.e. the first user's viewing direction 18 and the camera viewing direction 20. Since the camera 14 cannot be placed directly in front of the user's eyes, a minimal displacement of the camera 14 and first user 24 will always be present.

As an advantage, the camera 14 provides a view to other users that shows the scenery in more or less the same perspective of the first user 24. However, unlike a camera that is head-worn by the first user, the provided view is much more stable and less shaky. As an effect, less image movement compensation is needed, while still providing a look through the eyes of the first user, e.g. the eyes of a physician.

The term "camera device" relates to a device that captures optical images e.g. as visible to the human eye. In an option, also cameras using light outside the visible range are provided, in addition or alternatively, such as cameras capturing ultralight and/or infrared images and translating them into visible image data.

The term "view tracking device" relates to detecting a viewing direction and field of view as seen by the first user and monitoring any change in that direction and field of view.

The term "viewing direction" relates to a direction of the user's field of view. As an example, the focus line of the eyes is used as a center line and a certain angular range around the focus line is also provided as the first user's field of view.

The term "live stream of images" relates to providing a sequence of current images with a rather small timely delay such that the second user closely follows what the first user is currently seeing.

The term "adaptable camera viewing direction" relates to the option to change the center line or main focus line of the image data used for providing the live streak to the user. The viewing direction can be adapted by an actual movement of the camera, or by only selecting a portion of the camera's image stream.

The term "second user" relates to another staff member than the actual first user. The terms first and second are used for differentiation of the two staff members. The terms do not reflect any hierarchical order or the like.

The camera is configured to provide the live stream of images to at least one second user. The second user is provided with a view as if viewing though the first user's eyes.

For example, the camera device 12 enhances remote collaboration where the other party can look over the shoulder of the physician, but without the need for the latter to actually wear a device.

In an example, the camera device 12 functions as a so-called third person collaboration camera. In another example, in addition or alternatively, the camera device 12 functions as a monitoring and documenting camera.

The first user's viewing direction 18 can also be referred to as first user's view.

The camera viewing direction 20 can also be referred to as camera view.

In an example, the camera device 12 comprises at least one camera 14.

In an example, the view tracking device comprises 16 at least one tracker.

In an example, the view tracking device 16 comprises at least one tracking unit that tracks the first user's viewing direction 18.

As an option, the camera device 12 is configured to adapt the camara viewing direction 20 such that the live stream of images provided by the camera device to the second user 26 at least partly shows what the first user is viewing.

The term "least partly shows" relates to an at least partly common overlap from what is shown in the camera image stream and what the first user actually sees. In an option, the images show at least half of the same context as what the first user sees. In an option, the images show approximately the same context as what the first user sees, i.e. a maximum of +/- 10% deviation, such as up to +/- 5% deviation.

As an option, a data processing unit 24 is provided that is configured to provide a control signal to adapt the camara viewing direction 20 to be aligned with the tracked first user's viewing direction 18.

In an option, the tracking device 16 provides the tracked viewing direction to the data processing unit 24. In an option, the tracking device 16 provides tracking data to the data processing unit 24 and the data processing unit 24 is configured to determine the first user's viewing direction 20 based on the tracking data.

As an option, the view tracking device 16 is camera-based and comprises at least one tracking camera.

The term "camera-based" relates to tracking the view by camera devices, i.e. by optical cameras and their provided image data.

In an option, the view tracking device 16 is radiation-based and comprises at least one radiation transmitter/receiver, e.g. applying electromagnetic radiation like radar or lidar.

In another option, the view tracking device 16 is tracker-based and comprises at least one tracker attached to the first user's head.

To observe the first user, in an example, one fixed camera is provided. In another example, several fixed cameras are provided. In a further example, one movable camera is provided. In another example, several movable cameras are provided.

As another option, provided in addition or alternatively to the an eye tracking unit, the view tracking device 16 comprises at least one head-tracker unit 38. The head-tracker unit 38 is configured to track a spatial position and spatial orientation of the first user's head for determining a possible viewing direction 18 of the first user.

The term "head-tracker unit" relates to tracking the head movement and motion of the user's head.

In an example, the head-tracker unit is camera-based. For example, a camera is observing the first user and the stream of images is used for detecting the first user's head to determine the first user's viewing direction.

The head tracking can be provided based on one head-tracking camera feed or based on a feed of several head-tracking cameras.

The head can be tracked in the image even from a distance, for example when using a camera with high resolution.

As an option, provided in addition or alternatively to the head tracking unit 38, the view tracking device 16 comprises at least one eye-tracker unit 40. The eye-tracker unit 40 is configured to track a spatial position and spatial orientation of the first user's pair of eyes for determining a possible viewing direction 18 of the first user.

As an option, a filtering unit (not shown in details) is provided for eye-tracking with filtering.

The term "eye-tracker unit" relates to tracking the eyes and the respective viewing direction and the changes thereof of the user's view.

In an example, the eye-tracker unit 40 comprise at least one eye-tracking device. In an option, two or more eye-tracking devices are provided.

In an example, the eye-tracker unit 40 is camera-based. For example, a camera is observing the first user and the stream of images is used for detecting the first user's eyes to determine the first user's viewing direction.

The eye tracking can be provided based on one eye-tracking camera feed or based on a feed of several eye-tracking cameras.

The eyes can be tracked in the image even from a distance, for example when using a camera with high resolution.

The eye-tracking, or head-tracking, with filtering provides the option of smoothing out the eye- or head- tracking movements before having the camera follow them.

Due to an eye-tracking by an external device, but with filtering, a more stabilized view is provided. Contrary to tracking eyes by a user's head worn device, the direct tracking avoids tracking a user's head movement as a basis, which would result in more shaky view even though the user actually looks at the same spot, since a user automatically compensates for his fast head motions by moving his eyes. Persons following the head-mounted camera feed on a screen cannot do so and can experience very jerky (sometimes even nausea-inducing) motions. By providing a direct eye-tracking with filtering and an external camera that so-to-speak mimics the user's view, and persons following the camera feed from the separate camera are provided with an improved view.

An external camera also provides the advantage of being able have different fields of view in order to not miss events in the periphery of the physician. A ceiling-mounted, or otherwise supported camera, in combination with eye tracking provides a good angle with sufficient resolution of what the user is looking at from a good angle.

In an example, a combination of head and eye tracking is provided. In an example, head tracking may be sufficient, but the camera orientation can be made more precise by utilizing eye tracking.

The head- or eye-tracker tracks what the physician is looking at.

As an option, the camera device 12 comprises at least one movable camera, as indicated with motion arrows 42.

The term "movable camera" relates to a camera that can change at least one of the group of focal distance, viewing angle, position and orientation.

In a simple and thus facilitated form, the camera device 12 comprises a rotatable mount arranged at e.g. a ceiling structure above an average standpoint for a surgeon.

In another rather simple form, the camera device 12 is mounted slidably on rails at e.g. the ceiling structure wherein the rails cover an average range of stand points taken by the surgeon.

As an option, the at least one movable camera is mounted to a movable structure comprising at least one of the group of a robotic arm and a movable X/Y-frame.

The term "robotic arm" relates to an arm-like device movably hold by a base and the camera being mounted to its other end. The arm can comprise one, two, three or more arm segments. The robotic arm may allow movement within a 3D range.

The term "movable X/Y-frame" relates to a grid of rails allowing movement within a plane, e.g. in a 2D range.

In an example, a grid of cameras is provided. In an option, a plurality of stationary cameras is provided. In another option, a plurality of movable cameras is provided. In a further option, movable and stationary cameras are combined.

As an option, the camera device comprises a plurality of fixed cameras.

The term "fixed cameras" relates to a camera that does not can change position and orientation. In an example, the fixed camera does also not change its focal distance. In a further example, the fixed camera does also not change its viewing angle.

For example, a grid of cameras is provided.

In an option, a remote connectivity solution for the camera feed is provided. As an example, this can be a custom solution or a pre-existing such as Reacts, Teams, Zoom, etc.

As an option, an algorithm is provided that translates the first user's viewing direction to an over-the-shoulder viewing position and vector direction for the provision of the live stream of images; the camera viewing direction is adapted to best match the over-the-shoulder viewing position and vector direction.

In an option, the data processing unit is configured to:
- translate the first user's viewing direction derived from data provided by the tracking device to adapt the camera device to provide an over-the-shoulder viewing position and vector direction for the provision of the live stream of images; and
- provide adapted camera control data such that the camera viewing direction is adapted to best match the over-the-shoulder viewing position and vector direction.

In an example, with a movable camera, this algorithm moves the camera to have a similar viewing position as the user, but offset, to the side, above, and behind the user.

In another example, with a fixed camera, this algorithm chooses the camera that is closest to having a similar viewing position as the user, but offset, to the side, above, and behind the user. It can also average motions and reduces strong jerks.

The algorithm can also comprise a buffer function that averages motions and reduces strong jerks by applying a threshold when determining a change in the first user's viewing direction. The threshold can relate to time and/or to spatial differences. In an example relating to time, the buffer function monitors if the first user is keeping the viewing direction after detecting a change in the first user's viewing direction before adapting the viewing direction of the camera. In an example relating to spatial differences, the buffer function monitors if the first user is changing the viewing direction for more than a threshold value after detecting a change in the first user's viewing direction before adapting the viewing direction of the camera.

As an option, for adapting the camara viewing direction 20, spatial boundaries are provided that limit the adaptation of the camera to provide the live stream of images to the defined boundaries of the scenery of interest.

The term "spatial boundaries" relates to defined virtual boundaries that limit the possible viewing radius for the camera feed. The spatial boundaries provide a spatial constraint for what is shown by the camera.

In an option, the data processing unit is configured to consider provided spatial boundaries that limit the adaptation of the camera to provide the live stream of images to the defined boundaries of the scenery of interest.

Geometrical constraints are provided to ensure that the at least one second user is provided with images showing the scenery of interest and not images of parts outside the scenery.

As another option, provided alternatively or in addition, for adapting the camara viewing direction 30, a time-out function is provided when the first user looks at a further image source within the operation room. Instead of the live stream of images, a feed of the image data of the further image source is provided to the second user.

The term "time-out function" relates to a replacement of the camera feed by referring directly to the respective other image data source in order to better show the data the first user is actually viewing and thus considering.

In an option, the data processing unit is configured to:
- consider a provided time-out function when the first user looks at a further image source within the operation room; and
- providing a feed of the image data of the further image source to the second user instead of the live stream of images.

Temporal constraints are provided to ensure that the at least one second user is provided with images showing the scenery of interest and not images of parts outside the scenery.

As an option, a focus of the first user's view is detected, and a focus indicator is projected overlaid to the live stream of images. Upon detecting a change in the viewing direction and thus a change of the focus, the focus indicator is adapted in its relative location first and the change of the camera viewing direction is adapted with a temporal delay.

The term "focus indicator" relates to providing some sort of visible symbol or indicator of the focus point of the first user. The focus indicator illustrates in an intuitive way to the second user where the first user is currently focusing his view, which focus is usually the part the first user is most interested in. The second user can thus follow the first user's attention, while the image remains at least for a predetermined phase, before the camera image is also changing.

In an option, the data processing unit is configured to:
- detect a focus of the first user's view and project a focus indicator overlaid to the live stream of images; and
- upon detecting a change in the viewing direction and thus a change of the focus, adapted the focus indicator in its relative location first and adapt the change of the camera viewing direction with a temporal delay.

As already indicated above, Fig. 1 also shows, an option, a medical visualization system 100 that comprises an example of the visualization arrangement 10 described above and a display arrangement 102 comprising at least one display device 104. The at least one display device is configured to present the live stream of images to at least one second user based on the provided data of the live stream of images.

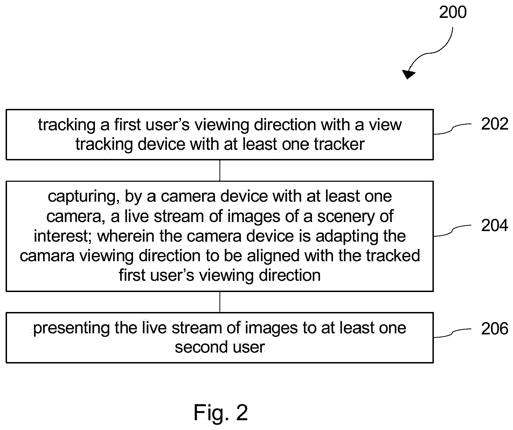

Fig. 2 shows basic steps of an example of a method 200 for providing additional visual information during a medical intervention. The method comprises the following steps:
- In a first step 202, a first user's viewing direction is tracked with a view tracking device having at least one tracker.
- In a second step 204, a live stream of images of a scenery of interest is captured by a camera device with at least one camera; the camera device is adapting the camara viewing direction to be aligned with the tracked first user's viewing direction; and
- In a third step 206, the live stream of images is presented to at least one second user.
A field of application are interventional procedures where it is important that others can see what the treating physician is seeing. It could however also be used in many other, non-medical collaboration situations.

The term "subject" may also be referred to as individual. The "subject" may further also be referred to as patient, although it is noted that this term does not indicate whether any illness or disease is actually present with the subject.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A visualization arrangement (10) for medical interventions, the arrangement comprising:
- a camera device (12) with at least one camera (14); and
- a view tracking device (16) with at least one tracker;
wherein the view tracking device is configured to track a first user's viewing direction (18);
wherein the camera device is configured to provide a live stream of images of a scenery of interest with an adaptable camera viewing direction (20);
wherein the camera device is configured to adapt the camera viewing direction to be aligned with the tracked first user's viewing direction; and
wherein the camera device is configured to provide the data of the live stream of images for presentation to a second user or more.

2. Arrangement according to claim 1, wherein the camera device is configured to adapt the camara viewing direction such that the live stream of images provided by the camera device to the second user at least partly shows what the first user is viewing.

3. Arrangement according to claim 1 or 2, wherein a data processing unit 24 is provided that is configured to provide a control signal to adapt the camara viewing direction to be aligned with the tracked first user's viewing direction.

4. Arrangement according to claim 1, 2 or 3, wherein the view tracking device is camera-based and comprises at least one tracking camera.

5. Arrangement according to one of the preceding claims, wherein the view tracking device comprises at least one head-tracker unit 38; and
wherein the head-tracker unit is configured to track a spatial position and spatial orientation of the first user's head for determining a possible viewing direction of the first user.

6. Arrangement according to one of the preceding claims, wherein the view tracking device comprises at least one eye-tracker unit 40; and
wherein the eye-tracker unit is configured to track a spatial position and spatial orientation of the first user's pair of eyes for determining a possible viewing direction of the first user; and
wherein a filtering unit is provided for eye-tracking with filtering.

7. Arrangement according to one of the preceding claims, wherein the camera device comprises at least one movable camera.

8. Arrangement according to claim 7, wherein the at least one movable camera is mounted to a movable structure comprising at least one of the group of:
i) a robotic arm; and
ii) a movable X/Y-frame.

9. Arrangement according to one of the preceding claims, wherein the camera device comprises a plurality of fixed cameras.

10. Arrangement according to one of the preceding claims, wherein an algorithm is provided that translates the first user's viewing direction to an over-the-shoulder viewing position and vector direction for the provision of the live stream of images; and
wherein the camera viewing direction is adapted to best match the over-the-shoulder viewing position and vector direction.

11. Arrangement according to one of the preceding claims, wherein, for adapting the camara viewing direction, spatial boundaries are provided that limit the adaptation of the camera to provide the live stream of images to the defined boundaries of the scenery of interest.

12. Arrangement according to one of the preceding claims, wherein, for adapting the camara viewing direction, a time-out function is provided when the first user looks at a further image source within the operation room; and
wherein, instead of the live stream of images, a feed of the image data of the further image source is provided to the second user.

13. Arrangement according to one of the preceding claims, wherein a focus of the first user's view is detected, and a focus indicator is projected overlaid to the live stream of images; and
wherein, upon detecting a change in the viewing direction and thus a change of the focus, the focus indicator is adapted in its relative location first and the change of the camera viewing direction is adapted with a temporal delay.

14. A medical visualization system (100), comprising:
- a visualization arrangement (10) according to one of the preceding claims; and
- a display arrangement (102) comprising at least one display device (104);
wherein the at least one display device is configured to present the live stream of images to at least one second user based on the provided data of the live stream of images.

15. A method (200) for providing additional visual information during a medical intervention, the method comprising:
- tracking (202) a first user's viewing direction with a view tracking device with at least one tracker;
- capturing (204), by a camera device with at least one camera, a live stream of images of a scenery of interest; wherein the camera device is adapting the camara viewing direction to be aligned with the tracked first user's viewing direction; and
- presenting (206) the live stream of images to at least one second user.
